(19) Europäisches Patentamt

European Patent Office

Office européen des brevets

(11) **EP 4 137 107 A1**

(12) **EUROPEAN PATENT APPLICATION**
published in accordance with Art. 153(4) EPC

(43) Date of publication:
**22.02.2023 Bulletin 2023/08**

(21) Application number: **21800019.8**

(22) Date of filing: **07.05.2021**

(51) International Patent Classification (IPC):
***A61F 13/15*** (2006.01)

(52) Cooperative Patent Classification (CPC):
**A61F 13/15**

(86) International application number:
**PCT/JP2021/017572**

(87) International publication number:
**WO 2021/225170 (11.11.2021 Gazette 2021/45)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.05.2020 JP 2020082675**

(71) Applicant: **Zuiko Corporation
Ibaraki-shi
Osaka 5670082 (JP)**

(72) Inventors:
• **UMEBAYASHI, Toyoshi
Settsu-shi, Osaka 566-0045 (JP)**
• **IKEDA, Natsuki
Settsu-shi, Osaka 566-0045 (JP)**

(74) Representative: **Müller-Boré & Partner
Patentanwälte PartG mbB
Friedenheimer Brücke 21
80639 München (DE)**

(54) **METHOD FOR MANUFACTURING WEARABLE ARTICLE**

(57) Provided is a method for manufacturing a wearable article with a capability to reduce a discrepancy between sealing areas on outward and homeward paths through which superposed parts of a continuous material are joined to each other when a wearable article that includes an absorptive body is manufactured. A method for manufacturing a wearable article includes: a superposing step of superposing a first part (W1) and a second part (W2) of a continuous material (W) on each other with an absorptive body (A) interposed between the first part (W1) and the second part (W2); a conveying step of winding the continuous material (W) including the first part (W1) and the second part (W2) superposed on each other around a rotary drum (5) and conveying the continuous material (W) with the absorptive body (A) put into a space (23) between two support faces (5b1, 5b2) on an outer peripheral surface (5b) of the rotary drum (5); and a joining step of moving a movable part (10) of a sealer unit (7) outward and homeward in a cross direction crossing a conveyance direction in which the continuous material (W) is conveyed, while the continuous material (W) with the absorptive body (A) put into the space (23) is conveyed by the rotary drum (5), to form liner sealing areas (S 1, S2) and thereby joining the first part (W1) and the second part (W2) to each other through the formed liner sealing areas.

FIG.5

## Description

## Technical Field

The present invention relates to a method for manufacturing a wearable article.

## Background Art

Conventionally, there have been various methods proposed for manufacturing wearable articles such as disposable diapers in succession.

For instance, a method for manufacturing a wearable article, described in Patent Literature 1, involves superposing a front body part and a back body part of a continuous material on each other, the front body part and the back body part making up a wearable article, and winding the continuous material including the front body part and the back body part superposed on each other around a rotary drum to convey the continuous material. The method also involves moving an anvil roller of a sealer unit outward and homeward along straight travel paths in a direction crossing a direction in which the continuous material is conveyed, with the sealer unit being rotated at a rate identical to that of the rotary drum, while the continuous material including the superposed front body part and back body part is conveyed by the rotary drum, and thereby joining the front body part and the back body part to each other through liner sealing areas along the outward-and-homeward travel paths in an area of either end of each wearable article in the continuous material.

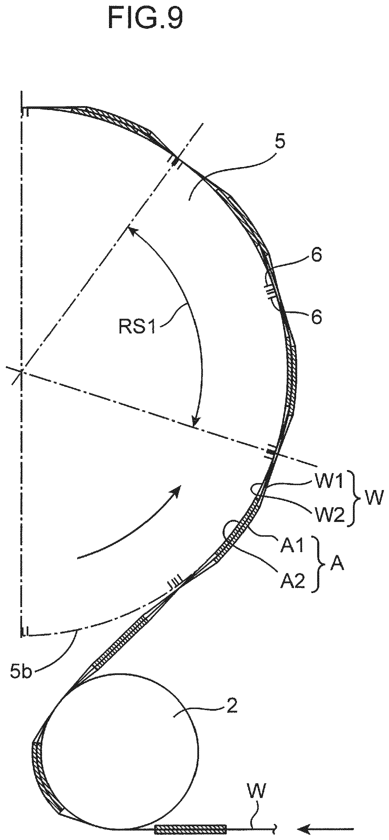

However, the method for manufacturing a wearable article, shown in Patent Literature 1, causes a difference in peripheral velocity between one of the front body part and the back body part disposed on an inner periphery side of the drum and the other body part disposed on an outer periphery side (for example, see a first part W1 and a second part W2 of a continuous material W in FIG. 9) when the continuous material, from which wearable articles are made, is wound around the rotary drum with the front body part and the back body part being superposed on each other.

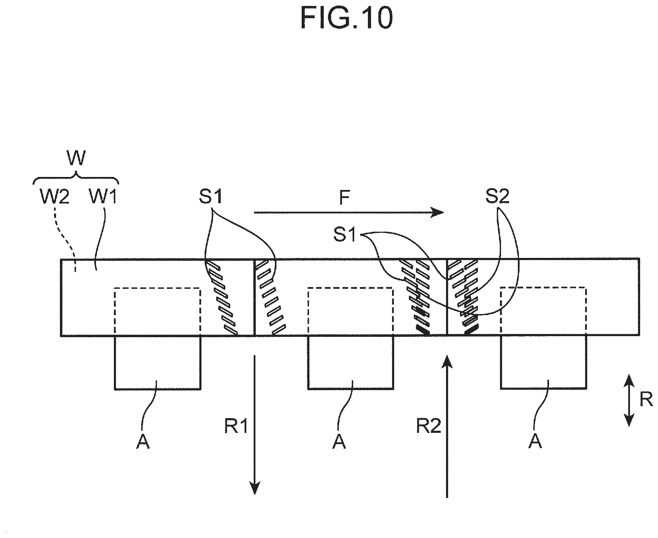

As a result, there is a problem of the occurrence of a discrepancy in sealing area between the outward and homeward paths due to the occurrence of a peripheral velocity difference between the sealer unit and either the front body or the back body (for example, see sealing areas S1, S2 in FIG. 10) when the parts of the continuous material are joined to each other by an outward-and-homeward movement of the sealer unit in the direction crossing the direction in which the continuous material is conveyed.

Such a discrepancy in sealing area has a dimension of 1 mm or more that is visible on a peripheral surface of an ordinarily-used rotary drum with a large diameter of approximately 2 m. The discrepancy is further noticeable especially for a wearable article such as an underpants-type diaper that includes a thick absorptive body due to an increased difference in peripheral velocity between the sealer unit and either the front body or the back body caused by the thickness of the absorptive body.

## Citation List

## Patent Literature

Patent Literature 1: WO 2015/098535 A

## Summary of Invention

It is an object of the present invention to provide a method for manufacturing a wearable article with a capability to reduce a discrepancy between sealing areas on outward and homeward paths through which superposed parts of a continuous material are joined to each other when a wearable article that includes an absorptive body is manufactured.

A method of the present invention for manufacturing a wearable article, accomplished to solve the above challenge, includes: a superposing step of superposing a first part and a second part of a wearable article on each other with an absorptive body interposed between the first part and the second part, the wearable article being included in a continuous material; a conveying step of winding the continuous material including the first part and the second part superposed on each other around an outer peripheral surface of a rotary drum, putting at least a part of the absorptive body into a space between two support faces that are separated from each other on the outer peripheral surface of the rotary drum in a rotation direction of the rotary drum, and conveying the continuous material with the at least part of the absorptive body put into the space by rotation of the rotary drum such that the first part and the second part are supported by the two support faces and the at least part of the absorptive body is disposed inside relative to the two support faces in a radial direction of the rotary drum; and a joining step of moving a movable part of a sealer unit outward and homeward in a cross direction crossing a conveyance direction in which the continuous material is conveyed, with the sealer unit being rotated at a rate identical to a rate of the rotary drum while the continuous material with the at least part of the absorptive body put into the space is conveyed by the rotation of the rotary drum, to form liner sealing areas on an outward path and a homeward path, respectively, and thereby joining the first part and the second part to each other through the formed liner sealing areas.

## Brief Description of Drawings

FIG. 1 is a process drawing for illustrating a method for manufacturing a disposable diaper as an example of a method for manufacturing a wearable article according to an embodiment of the present invention.

FIG. 2 is a schematic elevation view showing a configuration of an ultrasonic welding device used to execute a joining step shown in FIG. 1.

FIG. 3 is a cross-sectional view taken along line III-III of FIG. 2.

FIG. 4 is a cross-sectional view taken along line IV-IV of FIG. 3.

FIG. 5 is an illustrative drawing showing a process in a method for manufacturing a wearable article according to an embodiment of the present invention, the process being executed to put absorptive bodies arranged at equal intervals on a continuous material into recesses constituting spaces formed in an outer peripheral surface of a rotary drum in a conveying step and join a first part and a second part of the continuous material together with each of the absorptive bodies put into each of the recesses in a joining step.

FIG. 6A is an enlarged cross-sectional view of the recess constituting a space formed in the outer peripheral surface of the rotary drum in FIG. 5.

FIG. 6B is an illustrative cross-sectional view showing a situation in which the absorptive body is put into the recess in FIG. 6A.

FIG. 7 is an illustrative drawing showing a joining step executed by the ultrasonic welding device of FIG. 2 to form outward and homeward sealing areas.

FIG. 8A is an enlarged cross-sectional view of a space constituted of a through-hole penetrating through a peripheral wall that forms an outer peripheral surface of a rotary drum in a shape of a cylindrical hollow body in a modification example of a method of the present invention for manufacturing a wearable article.

FIG. 8B is an illustrative cross-sectional view showing a situation in which an absorptive body is put into the through-hole in FIG. 8A in a modification example of a method of the present invention for manufacturing a wearable article.

FIG. 9 is a drawing showing a situation in which a method for manufacturing a wearable article using a rotary drum without a space in an outer peripheral surface in a comparative example of the present invention causes a difference in peripheral velocity between a first part and a second part superposed on each other of a continuous material on the rotary drum and the peripheral velocity difference results in a discrepancy between the first part and the second part.

FIG. 10 is a drawing showing a discrepancy in sealing area between outward and homeward paths when outward and homeward sealing areas are formed over the rotary drum of FIG. 9.

## Description of Embodiments

Hereinafter, a preferred embodiment of the present invention will be described in detail with reference to the attached drawings.

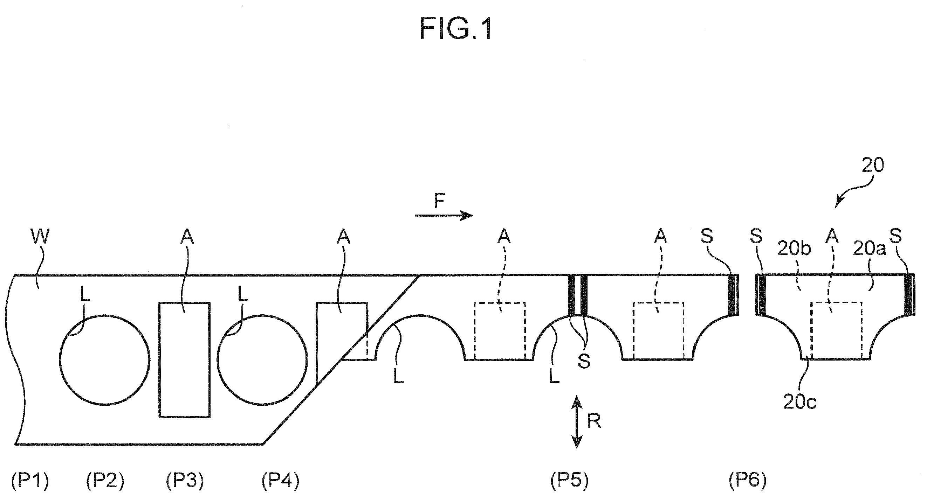

A wearable article 20 manufactured by a manufacturing method of the present invention is, for example, a wearable article such as a disposable diaper or underpants that can cover a lower half of one's body, as shown in FIG. 1, and includes a front body 20a disposed on an abdomen of a wearer wearing the wearable article, a back body 20b disposed on buttocks of the wearer, and a crotch 20c extending from the front body 20a to the back body 20b through an area between the tops of the wearer's legs.

Both side edges of the front body 20a and both side edges of the back body 20b are joined together such that the coupled front body 20a and back body 20b form an annular shape. Specifically, the front and the back bodies are welded to each other through two sealing areas S by ultrasonic welding.

A basic procedure for manufacturing the wearable article 20 is as described below.

<Conveying step P1>

In a conveying step P1, a long sheet-shaped continuous material W extending in a specific direction is conveyed along a lengthwise direction of the material (a conveyance direction F). A description is given hereinafter with a direction in which the continuous material W is carried being defined as a lateral direction and a direction orthogonal to the lateral direction in FIG. 1 as a longitudinal direction.

The continuous material W includes an inside sheet facing a body surface of the wearer wearing the wearable article, an outside sheet facing outward from the wearer wearing the wearable article, and an elastic member that is put between the inside sheet and the outside sheet and that is elastic at least in the conveyance direction F. The outside sheet is made of a nonwoven sheet permeable to liquid and/or a mesh sheet, or a polyethylene film, a polypropylene film, or a heat seal resin-made nonwoven fabric that is waterproof and breathable.

<Leg hole forming step P2>

In a leg hole forming step P2, a leg hole L is formed at a middle in the longitudinal direction of the continuous material W.

A region between the two leg holes L in the continuous material W is a part corresponding to the crotch 20c. Places on both sides of the part corresponding to the crotch 20c in the longitudinal direction of the continuous material W are parts corresponding to the front body 20a and the back body 20b, respectively.

In other words, the conveying step P1 and the leg hole forming step P2 correspond to a preparation step for preparing a continuous material in which constituent elements are consecutive in the lateral direction. The front body 20a and the back body 20b are coupled through the crotch 20c in the longitudinal direction to make up each of the constituent elements.

<Absorptive body joining step P3>

In an absorptive body joining step P3, an absorptive body A is joined to a place between the two leg holes L in the continuous material W.

The absorptive body A includes a permeable sheet permeable to liquid, a waterproof sheet that is waterproof and breathable, and an absorptive core put between the permeable sheet and the waterproof sheet. The permeable sheet is made of a nonwoven sheet and/or a mesh sheet which are permeable to liquid. The waterproof sheet is made of a polyethylene film, a polypropylene film, or a nonwoven fabric that is waterproof and breathable. The absorptive core is shaped by arranging crushed pulp or crushed pulp mixed with a super absorbent polymer in layers.

<Superposing step P4>

In a superposing step P4, the continuous material W on which the absorptive body A is placed is folded in half in the longitudinal direction (in other words, at a middle position in a widthwise direction (the longitudinal direction) of the continuous material W). As a result, the part corresponding to the front body 20a and the part corresponding to the back body 20b of the continuous material W are superposed on each other.

(Joining step P5)

In a joining step P5, a part corresponding to a side edge of the front body 20a and a part corresponding to a side edge of the back body 20b of the continuous material W folded in half are joined together by ultrasonic welding.

Specifically, in the joining step P5, the parts at two places separated by a cutting zone that is set in advance as a zone to be cut in a cutting step P6 described later of the continuous material W are simultaneously joined together by ultrasonic welding to form liner sealing areas S extending in a cross direction R that crosses (that is orthogonal to, in the present embodiment) the conveyance direction F.

The two sealing areas S are formed on the parts corresponding to the side edges of the front body 20a and on the parts corresponding to the side edges of the back body 20b.

(Cutting step P6)

In the cutting step P6, the continuous material W is cut along a cutting line extending longitudinally between the two sealing areas S formed in the joining step P5. As a result, the continuous material W is cut on a wearable article by wearable article basis.

<Description of ultrasonic welding device 1>

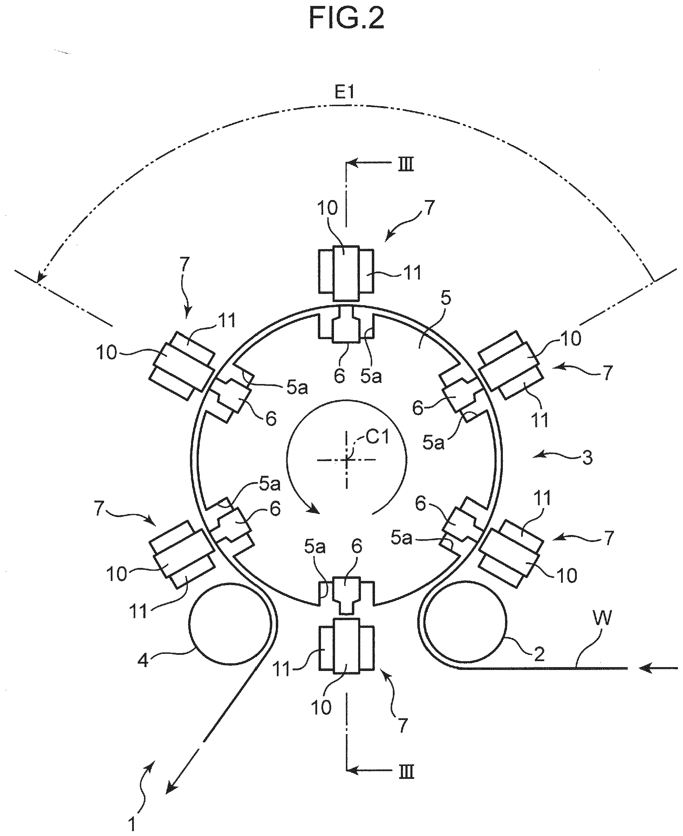

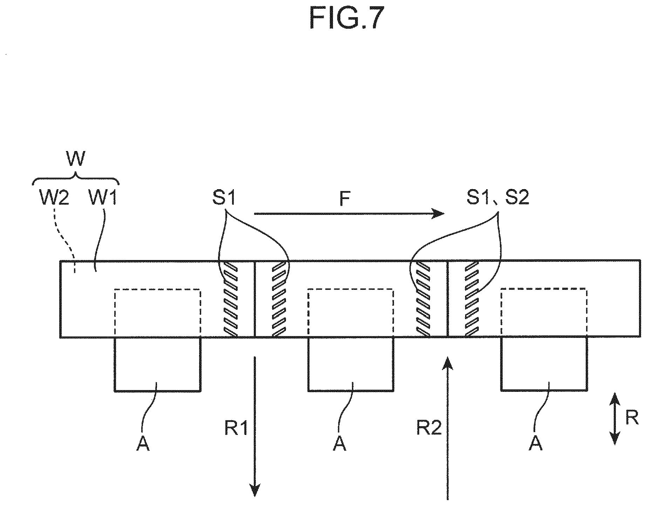

A joining device used to execute the joining step P5 is configured to move a movable part of a sealer unit outward and homeward in a cross direction R crossing a conveyance direction F in which the continuous material W is conveyed, while the continuous material W is conveyed by the rotation of the rotary drum 5, to form liner sealing areas S1, S2 shown in FIG. 7 on an outward path R1 and a homeward path R2, respectively, and join the first part W1 and the second part W2 to each other through the formed liner sealing areas in areas corresponding to both sides of the wearable article 20. For instance, an ultrasonic welding device 1 shown in FIG. 2 is used to execute the joining step P5.

The ultrasonic welding device 1 shown in FIG. 2 includes a bring-in anvil roller 2 to bring in the continuous material W folded in half in the superposing step P4, a welding drum unit 3 to weld the continuous material W brought in by the bring-in anvil roller 2, and a bring-out anvil roller 4 to bring out the continuous material W welded by the welding drum unit 3.

The welding drum unit 3 includes a rotary drum 5 to hold the continuous material W brought in by the bring-in anvil roller 2 and six ultrasonic sealer units 7 serving as a sealer unit for joining of the continuous material W to ultrasonically weld the continuous material W.

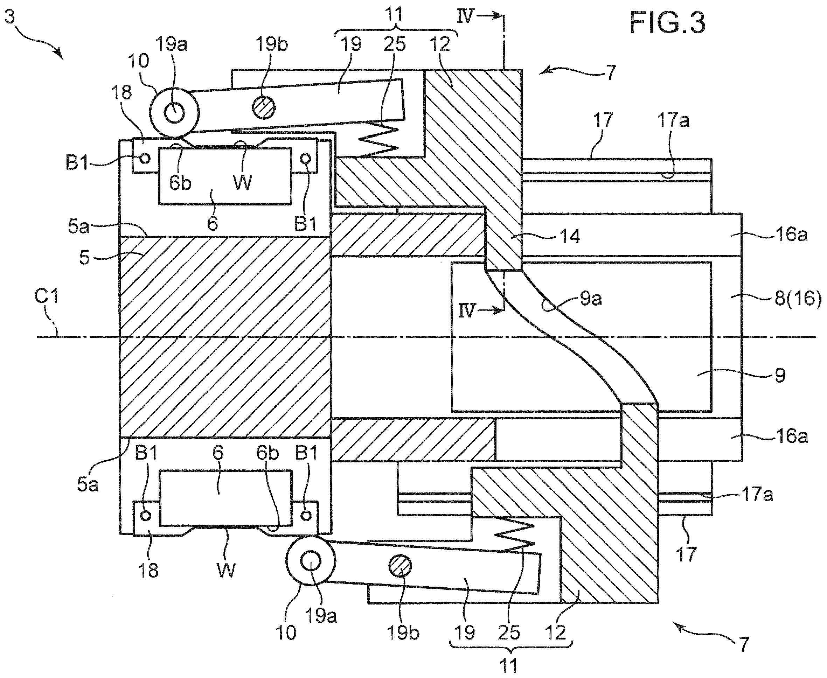

The ultrasonic sealer units 7, as shown in FIGS. 2 to 3, include six ultrasonic horns 6 disposed on the rotary drum 5, anvil rollers 10 (movable parts) to weld the continuous material W in conjunction with the ultrasonic horns 6, and holding members 11 to hold the respective anvil rollers 10 in such a way as to be movable relative to the rotary drum 5 along a rotation center C1 (i.e., movable in a superior-inferior direction in FIG. 3).

The welding drum unit 3 also includes an anvil holding drum 8 (see FIG. 3) in a shape of a tube to hold the holding members 11, a cam drum 9 (see FIG. 3) disposed inside the anvil holding drum 8, and six pressed members 18 (see FIG. 3) that are adjacent to the respective ultrasonic horns 6 and fixed to the rotary drum 5.

As shown in FIGS. 2 to 3, the rotary drum 5 is able to rotate on the rotation center C1 with the continuous material W being held on an outer peripheral surface of the rotary drum. Six recessed grooves 5a are formed around the rotation center C1 at equal intervals on the rotary drum 5. The recessed grooves 5a are opened outward from the rotary drum 5, extending along the rotation center C1.

The ultrasonic horns 6 give ultrasonic vibration to the continuous material W held by the rotary drum 5. The ultrasonic horns 6 each have an output side end 6b to output the ultrasonic vibration.

The ultrasonic horn 6, as shown in FIGS. 2 to 3, is disposed in the recessed groove 5a so as to come into contact with the continuous material W held by the rotary drum 5 from inside.

As shown in FIGS. 3 to 4, the holding member 11 includes a holding member body 12 attached to the anvil holding drum 8 so as to be movable along the rotation center C1, a holding lever 19 that is attached to the holding member body 12 so as to be pivotable around a pivot 19b and that holds the anvil roller 10 such that the anvil roller is rotatable on a rotation axis 19a, and a push member 25 that pushes the holding lever 19 in such a direction that the anvil roller 10 moves toward the ultrasonic horn 6.

The rotation axis 19a and the pivot 19b are axes each extending in a direction orthogonal to a plane including the rotation center C1 and the ultrasonic sealer units 7 (a direction orthogonal to a piece of paper of FIG. 3). The rotation axis 19a is disposed on a distal end of the holding lever 19, whereas the pivot 19b is disposed on a middle of the holding lever 19.

Thus, the anvil roller 10 is able to come into contact with and roll over the continuous material W in response to a movement of the holding member 11 along the rotation center C1 and is able to move toward or away from the continuous material W (the ultrasonic horn 6) in a radial direction of the rotary drum 5 in response to a pivot of the holding lever 19.

The push member 25 pushes a proximal end of the holding lever 19 with respect to the holding member body 12 in a direction in which the proximal end moves away from the rotation center C1 and thereby pushes the anvil roller 10 in a direction in which the anvil roller 10 moves toward the ultrasonic horn 6.

The push member 25, the holding lever 19, and the pivot 19b correspond to a push mechanism designed to push the anvil roller 10 toward the ultrasonic horn 6 such that the ultrasonic horn 6 and the anvil roller 10 move toward each other.

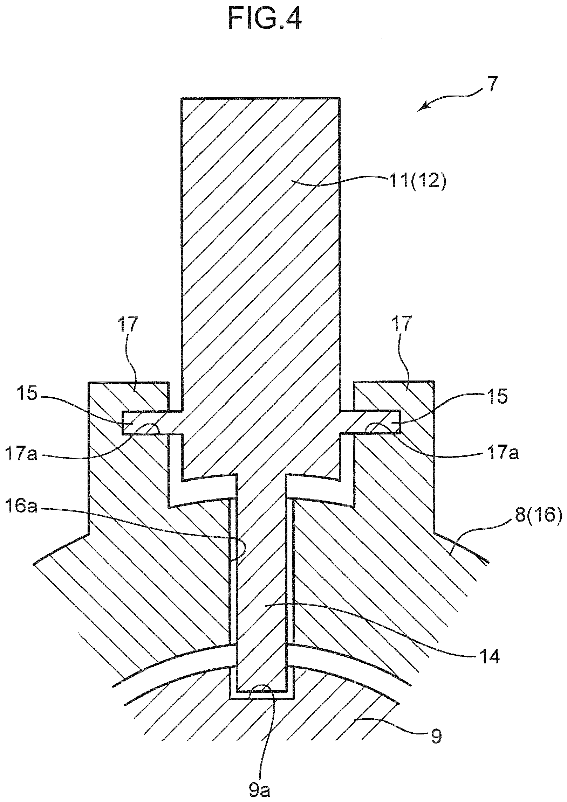

The holding member body 12 has a cam protrusion 14 extending toward the rotation center C1 and a pair of engagement protrusions 15 protruding in opposite directions along a direction (a crosswise direction in FIG. 4) orthogonal to both the cam protrusion 14 and the rotation center C1 and extending along the rotation center C1.

The holding member body 12 is disposed between a pair of rails 17 that stand on an outer peripheral surface of the anvil holding drum 8. The rails 17 each have an engagement groove 17a being opened to the opposite rail 17 side and extending along the rotation center C1. The engagement protrusions 15 of the holding member body 12 are in engagement with the respective engagement grooves 17a such that the holding member is movable relative to the anvil holding drum 8 along the rotation center C1.

The tubular anvil holding drum 8 includes a slit 16a penetrating through a peripheral wall of the anvil holding drum and extending along the rotation center C1.

The cam protrusion 14 of the holding member body 12 is inserted into the anvil holding drum 8 through the slit 16a.

The cam drum 9 is disposed inside the anvil holding drum 8, and a cam groove 9a is formed on an outer peripheral surface of the cam drum 9. A distal end of the cam protrusion 14 is inserted into the cam groove 9a. The cam groove 9a guides the cam protrusion 14 such that each ultrasonic sealer unit 7 moves along the rotation center C1 in response to rotation of the anvil holding drum 8 relative to the cam drum 9.

The rotary drum 5 and the anvil holding drum 8 are fastened to each other and rotate on the rotation center C1 in an integral manner. Meanwhile, a rotational position of the cam drum 9 is fixed regardless of the rotation of the rotary drum 5 and the anvil holding drum 8. Thus, the holding member body 12 moves along the rotation center C1 in response to the rotation of the rotary drum 5 and the anvil holding drum 8 on the rotation center C1.

Specifically, the anvil roller 10 and the holding member 11 at a lowest place in FIGS. 2 and 3 is disposed at a place separated from the continuous material W held by the rotary drum 5 in plan view. The anvil roller 10 and the holding member 11 in this state move toward the continuous material W along the rotation center C1 in response to counterclockwise rotation of the rotary drum 5 in FIG. 2.

In a course of displacement of the anvil roller 10 and the holding member 11 to a highest place in FIGS. 2 and 3, the anvil roller 10 crosses the continuous material W, and the anvil roller 10 at the highest place in FIGS. 2 and 3 is disposed at a place separated from the continuous material W held by the rotary drum 5 in plan view. The anvil roller 10 in this state crosses the continuous material W again in response to further counterclockwise rotation of the rotary drum 5 and returns to a position of the anvil roller 10 at the lowest place in FIGS. 2 and 3.

In other words, in a range E1 in FIG. 2, the anvil roller 10 of the ultrasonic welding device 1 moves outward and homeward on the continuous material W, and during this outward-and-homeward movement, the continuous material W is welded at parts of the sealing areas S. More specifically, the anvil rollers 10 positioned in a range outside the range E1 in FIG. 2 are at places separated from the continuous material W in plan view. When the anvil rollers 10 come in the range E1, the anvil rollers 10 move successively to a place that overlaps the continuous material W in plan view.

<Manufacturing method in present embodiment>

A method of the present embodiment for manufacturing the wearable article 20 is implemented through the following procedure using the ultrasonic welding device 1 described above.

Specifically, the method of the present embodiment for manufacturing a wearable article 20 includes:

(i) a superposing step (see the superposing step P4 in FIG. 1) of superposing a first part W1 and a second part W2 of a wearable article 20 on each other with an absorptive body A interposed between the first part W1 and the second part W2, the wearable article 20 being included in a continuous material W, the first part W1 and the second part W2 being corresponding to a front body and a back body respectively of the wearable article 20;

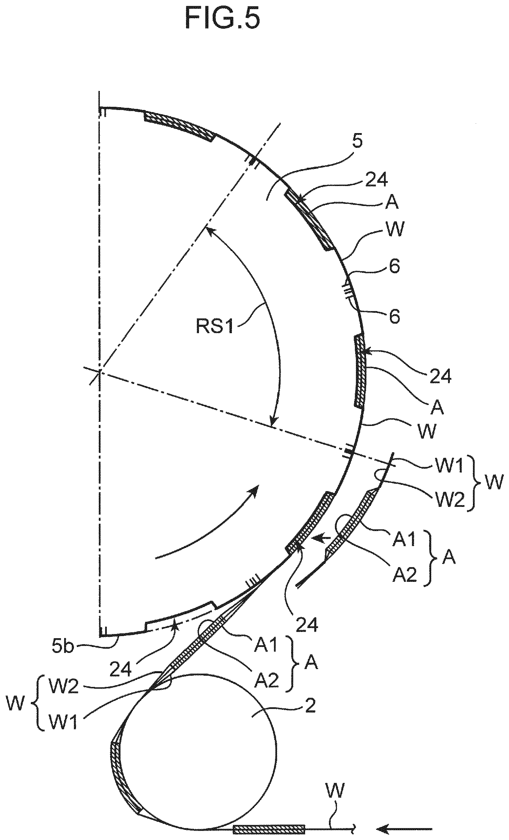

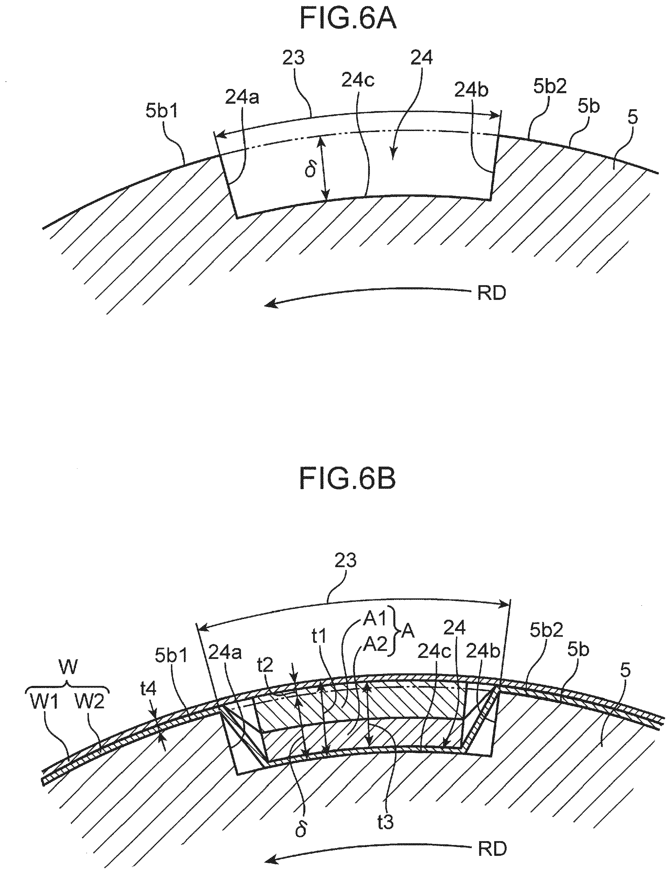

(ii) a conveying step (in other words, a conveying step between the superposing step P4 and the joining step P5 in FIG. 1) of winding the continuous material W including the first part W 1 and the second part W2 superposed on each other around an outer peripheral surface 5b of a rotary drum 5, as shown in FIGS. 5, 6A, and 6B, putting at least a part (in FIGS. 5 and 6B(b), a whole) of the absorptive body A into a recess 24 constituting a space 23 between two support faces 5b1, 5b2 that are separated from each other on the outer peripheral surface 5b of the rotary drum 5 in a rotation direction RD of the rotary drum 5, and conveying the continuous material W with the at least part of the absorptive body A put into the recess 24 by rotation of the rotary drum 5 such that the first part W1 and the second part W2 are supported by the two support faces 5b 1, 5b2 and the at least part of the absorptive body A is disposed inside relative to the two support faces 5b 1, 5b2 in a radial direction of the rotary drum 5; and

(iii) a joining step (the joining step P5 in FIG. 1) of moving an anvil roller 10 that is a movable part of each ultrasonic sealer unit 7 in FIGS. 2 to 3 outward and homeward in a cross direction R crossing a conveyance direction F in which the continuous material W is conveyed, with each of the ultrasonic sealer units 7 being rotated at a rate identical to a rate of the rotary drum 5 (for example, while the rotary drum 5 in FIG. 5 is rotated in a predetermined angular range RS1), while the continuous material W with the at least part of the absorptive body A put into the recess 24 is conveyed by the rotation of the rotary drum 5, to form liner sealing areas S1, S2 shown in FIG. 7 on an outward path R1 and a homeward path R2, respectively, and thereby joining the first part W1 and the second part W2 to each other (ultrasonic welding in the present embodiment) through the formed liner sealing areas in areas corresponding to both sides of the wearable article 20.

In an example shown in FIGS. 6A and 6B, the space 23 is constituted of the recess 24. However, as in a modification example (A) described later, the space 23 may be constituted of a through-hole 26.

The absorptive body A shown in FIGS. 5 and 6B(b) is folded in half (in other words, a first part A1 and a second part A2 are superposed on each other) since the first part W1 and the second part W2 of the continuous material W are superposed on each other in the superposing step (P4 in FIG. 1).

As shown in FIG. 6B, while the absorptive body A is put in the recess 24, a portion of the second part W2 of the continuous material W that is in contact with the absorptive body A is put between the absorptive body A and a bottom of the recess 24, extending along the bottom of the recess 24. A portion of the second part W2 that is not in contact with the absorptive body A is stretched obliquely from the outer peripheral surface 5b of the rotary drum 5 to the bottom of the recess 24. Meanwhile, the first part W1 of the continuous material W extends in a substantially arc shape along an upper surface of the absorptive body A.

In (ii), in the conveying step, the whole absorptive body A is put into the recess 24 formed in the outer peripheral surface 5b of the rotary drum 5, and in the joining step, the first part W1 and the second part W2 are joined together with the whole absorptive body A being put in the recess 24 to reduce a peripheral velocity difference between the ultrasonic sealer unit 7 and either the first part W1 or the second part W2.

Here, as shown in FIGS. 6A and 6B, a description is given of an amount of the absorptive body A and the continuous material W protruding from the outer peripheral surface 5b when the whole absorptive body A and the second part W2 on an inner periphery side of the continuous material W are put in the recess 24 with a depth $\delta$ formed in the outer peripheral surface 5b of the rotary drum 5.

Specifically, the following relationship (Equation 1) is satisfied:

$$t2 = t1 - \delta \qquad (1)$$

where t1 is a total thickness of the absorptive body A and the first part W1 and the second part W2 of the continuous material W; and t2 is an amount of the absorptive body A and the continuous material W overflowing the $\delta$-deep recess 24 and protruding from the outer peripheral surface 5b of the rotary drum 5. The depth $\delta$ of the recess 24 is a distance from the bottom 24c of the recess 24 to the outer peripheral surface 5b of the rotary drum 5.

When the depth $\delta$ of the recess 24 is greater than or equal to an overall thickness t3 of the absorptive body A, the protrusion amount t2 is equal to a total thickness t4 of the first part W1 and the second part W2 of the continuous material W over the outer peripheral surface 5b of the rotary drum 5. Thus, regarding the first part W1 on an outer periphery side, the amount of relative protrusion (raised amount) is 0 at a place at which the absorptive body A is present as compared with another place at which the absorptive body is not present.

As shown in FIGS. 6A and 6B, the recess 24 has a pair of side walls 24a, 24b that are separated from each other in the rotation direction RD of the rotary drum 5. Hence, if a distance between the pair of the side walls 24a 24b is set closer to a width of the absorptive body A (a width in the rotation direction RD), the absorptive body A put into the recess 24 can be held by the pair of the side walls 24a, 24b in the conveying step in (ii) and the joining step in (iii).

The movable part of the ultrasonic sealer unit 7 shown in FIGS. 2 to 3 may be any one of the anvil roller 10 and the ultrasonic horn 6.

Regarding the first part W1 corresponding to the front body and the second part W2 corresponding to the back body of the continuous material W, the continuous material is wound around the rotary drum 5 such that the first part W1 is disposed on an outer periphery side of the second part W2 in FIG. 5. However, they may be disposed in reverse, in other words, the continuous material may be wound around the rotary drum 5 such that the second part W2 is disposed on an outer periphery side of the first part W1.

(Characteristics of present embodiment)

(1) The method of the embodiment for manufacturing the wearable article 20 as described above includes: a superposing step of superposing a first part W1 and a second part W2 of a wearable article 20 on each other with an absorptive body A interposed between the first part W1 and the second part W2, the wearable article 20 being included in a continuous material W, the first part W1 and the second part W2 corresponding to a front body and a back body respectively of the wearable article 20; a conveying step of winding the continuous material W including the first part W1 and the second part W2 superposed on each other around an outer peripheral surface 5b of a rotary drum 5, as shown in FIGS. 5, 6A, and 6B, putting at least a part (in FIGS. 5 and 6B, a whole) of the absorptive body A into a recess 24 constituting a space 23 between two support faces 5b1, 5b2 that are separated from each other on the outer peripheral surface 5b of the rotary drum 5 in a rotation direction RD of the rotary drum 5, and conveying the continuous material W with the at least part of the absorptive body A put into the recess 24 by rotation of the rotary drum 5 such that the first part W1 and the second part W2 are supported by the two support faces 5b 1, 5b2 and the at least part of the absorptive body A is disposed inside relative to the two support faces 5b1 5b2 in a radial direction of the rotary drum 5; and a joining step of moving an anvil roller 10 that is a movable part of each ultrasonic sealer unit 7 in FIGS. 2 to 3 outward and homeward in a cross direction R crossing a conveyance direction F in which the continuous material W is conveyed, with each of the ultrasonic sealer units 7 being rotated at a rate identical to a rate of the rotary drum 5, while the continuous material W with the at least part of the absorptive body A put into the recess 24 is conveyed by the rotation of the rotary drum 5, to form liner sealing areas S1, S2 shown in FIG. 7 on an outward path R1 and a homeward path R2, respectively, and thereby joining the first part W1 and the second part W2 to each other through the formed liner sealing areas in areas corresponding to both sides of the wearable article 20.

In the manufacturing method, in the superposing step, the first part W1 corresponding to the front body and the second part W2 corresponding to the back body of the wearable article included in the continuous material W are superposed on each other with the absorptive body A interposed between the first part W1 and the second part W2, and in the conveying step, with the at least part of the absorptive body A being put into the recess 24 constituting the space 23 formed between the two support faces 5b1, 5b2 on the outer peripheral surface 5b of the rotary drum 5, the continuous material W including the first part W1 and the second part W2 superposed on each other is wound around the outer peripheral surface 5b of the rotary drum 5 and is conveyed by rotation of the rotary drum 5. Then, in the joining step, the first part W1 and the second part W2 are joined together with the at least part of the absorptive body A being put into the recess 24 (the space 23) such that the at least part of the absorptive body A is disposed inside relative to the two support faces 5b1, 5b2 in the radial direction of the rotary drum 5. Thus, in the joining step, the at least part of the absorptive body A is put into the recess 24 (the space 23). This reduces the amount of protrusion (raised amount) of either the first part W1 or the second part W2 (in the present embodiment, the first part W1 on the outer periphery side) from the outer peripheral surface 5b of the rotary drum 5 caused by the thickness of the absorptive body A. As a result, a peripheral velocity difference between the ultrasonic sealer unit 7 and either the first part W1 or the second part W2 can be reduced. This makes it possible to reduce a discrepancy between the sealing areas S1, S2 on the outward and homeward paths through which the superposed parts of the continuous material W are joined to each other.

In a comparative example, in a method for manufacturing a wearable article 20 using a conventional rotary drum 5 without a space 23, such as the recess 24, formed in the outer peripheral surface 5b, a peripheral velocity difference is made between the second part W2 disposed on the inner periphery side and the first part W1 disposed on the outer periphery side of the rotary drum 5 due to the thickness of the absorptive body A as shown in FIG. 9 when the continuous material W including the first part W1 and the second part W2 superposed on each other is wound around the rotary drum 5. This, as shown in FIG. 10, results in a problem of the occurrence of a discrepancy between the sealing areas S1, S2 on the outward path R1 and the homeward path R2 when the superposed parts are joined to each other by an outward-and-homeward movement of the ultrasonic sealer unit 7 in the cross direction R crossing the conveyance direction F for the continuous material W. Hence, it is clear that the conveying step and the joining step executed with the at least part of the absorptive body A being put into the space 23 such as the recess 24 in the outer peripheral surface 5b of the rotary drum 5 as in the above embodiment contribute to the effect of reducing a discrepancy between the outward and homeward sealing areas S1, S2 through which the superposed parts of the continuous material W are joined to each other.

(2) Preferably, in the method of the present embodiment for manufacturing the wearable article 20, in the conveying step, the whole absorptive body A is put into the space 23 such as the recess 24 formed in the outer peripheral surface 5b of the rotary drum 5, and in the joining step, the first part W1 and the second part W2 are joined together with the whole absorptive body A being put in the space 23 such as the recess 24.

In this case, in the joining step, the whole absorptive body A is put into the space 23 such as the recess 24 in the outer peripheral surface 5b of the rotary drum 5. This offsets the amount of protrusion of either the first part W1 or the second part W2 (in the present embodiment, the first part W1 on the outer periphery side) from the outer peripheral surface 5b of the rotary drum 5 caused by the thickness of the absorptive body A. As a result, a peripheral velocity difference between the ultrasonic sealer unit 7 and either the first part W1 or the second part W2 due to the thickness of the absorptive body A can be offset. This makes it possible to reliably reduce a discrepancy between the sealing areas S1, S2 on the outward and homeward paths through which the superposed parts of the continuous material W are joined to each other.

(3) Preferably, in the method of the present embodiment for manufacturing the wearable article 20, the depth δ of the recess 24 constituting the space 23 shown in FIGS. 6A and 6B is greater than or equal to the thickness t3 of the absorptive body A. In this case, in the joining step, with the whole absorptive body A being reliably put into the recess 24 in the outer peripheral surface 5b of the rotary drum 5 and the peripheral velocity difference between the ultrasonic sealer unit 7 and either the first part W1 or the second part W2 being reliably offset, the superposed parts can be joined together. This makes it possible to reduce a discrepancy between the sealing areas S1, S2 with increased reliability.

(4) In the method of the present embodiment for manufacturing the wearable article 20, the recess 24 constituting the space 23 may have a pair of side walls 24a, 24b that are separated from each other in the rotation direction RD of the rotary drum 5, and the absorptive body A put into the recess 24 may be held by the pair of the side walls 24a, 24b in the conveying step and the joining step.

In the manufacturing method, if the absorptive body A put into the recess 24 is held by the pair of the side walls 24a, 24b of the recess 24 in the conveying step and the joining step, the peripheral velocity difference between the ultrasonic sealer unit 7 and either the first part W1 or the second part W2 can be reduced to an increased extent. This makes it possible to further reduce a discrepancy between the sealing areas S1, S2 on the outward and homeward paths through which the superposed parts of the continuous material W are joined to each other.

(Modification examples)

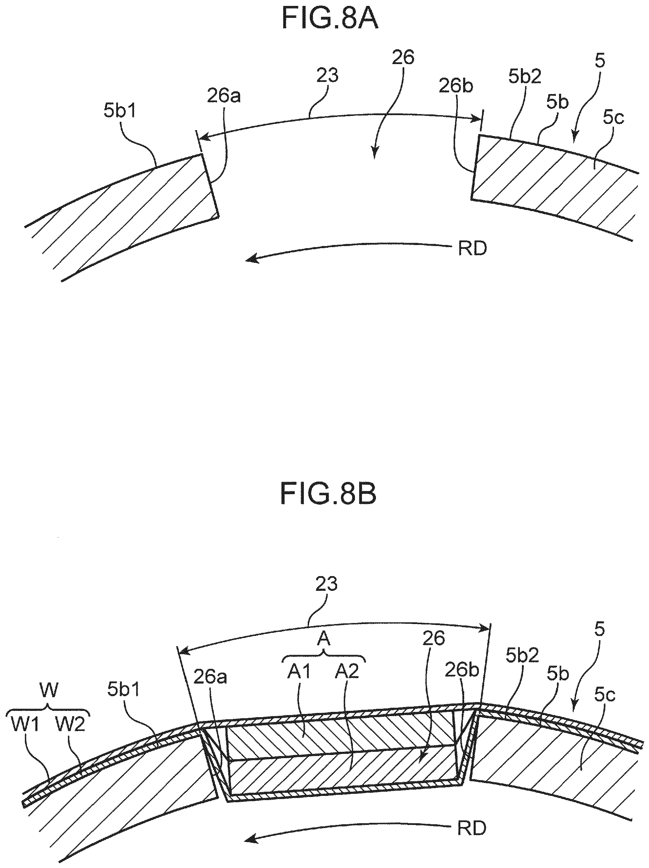

(A) The space 23 of the rotary drum 5 described above is constituted of the recess 24 that has the bottom 24c. However, the present invention should not be limited to this example. In a modification example of the present invention, as shown in FIGS. 8A and 8B, if the rotary drum 5 is in a shape of a cylindrical hollow body, the space 23 may be constituted of a through-hole 26 penetrating through a peripheral wall 5c that forms the outer peripheral surface 5b of the rotary drum 5.

As shown in FIG. 8B, while the absorptive body A is put in the through-hole 26, a portion of the second part W2 of the continuous material W that is in contact with the absorptive body A extends substantially linearly along an undersurface of the absorptive body A. A portion of the second part W2 that is not in contact with the absorptive body A is stretched obliquely from the outer peripheral surface 5b of the rotary drum 5 to the undersurface of the absorptive body A. Meanwhile, the first part W1 of the continuous material W extends substantially linearly along the upper surface of the absorptive body A.

In such a modification example shown in FIGS. 8A and 8B, the space 23 between two support faces 5b1, 5b2 on the outer peripheral surface 5b of the rotary drum 5 is constituted of the through-hole 26 penetrating through the peripheral wall 5c that forms the outer peripheral surface 5b of the rotary drum 5. Thus, in the joining step, irrespective of the thickness of the absorptive body A, with the whole absorptive body A being reliably put into the through-hole 26 formed in the outer peripheral surface 5b of the rotary drum 5 and the peripheral velocity difference between the ultrasonic sealer unit 7 and either the first part W1 or the second part W2 being reliably offset, the superposed parts can be joined together. This makes it possible to reliably reduce a discrepancy between the sealing areas S1, S2 irrespective of the thickness of the absorptive body A. As a result, the scope of application of this manufacturing method can be widened.

In the method of the modification example (A) for manufacturing the wearable article 20, in a similar way to the manufacturing method of the above embodiment, the space 23 constituted of the through-hole 26 may have a pair of side walls 26a, 26b that are separated from each other in the rotation direction RD of the rotary drum 5, and the absorptive body A put into the through-hole 26 may be held by the pair of the side walls 26a, 26b in the conveying step and the joining step.

In the manufacturing method of the modification example (A), in a similar vein, if the absorptive body A put into the through-hole 26 is held by the pair of the side walls 26a, 26b of the through-hole 26 in the conveying step and the joining step, the peripheral velocity difference between the ultrasonic sealer unit 7 and either the first part W1 or the second part W2 can be reduced to an increased extent. This makes it possible to further reduce a discrepancy between the sealing areas S1, S2 on the outward and homeward paths through which the superposed parts of the continuous material W are joined to each other.

(B) In the embodiment described above, the method for manufacturing a wearable article such as a disposable diaper that can cover a lower half of one's body has been described to give an example of a method for manufacturing a wearable article that includes an absorptive body. However, the manufacturing method of the present invention can be widely applied to the manufacture of any wearable article that includes an absorptive body. For instance, the manufacturing method of the present invention can be applied to a pouch-shaped wearable article such as a sanitary pad that is manufactured by superposing a first part and a second part of a continuous material on each other with an absorptive body put between the first part and the second part and thereby joining the first part and the second part together.

<Summary of embodiments>

The embodiments are summarized as follows.

A method for manufacturing a wearable article, according to the embodiments described above, includes: a superposing step of superposing a first part and a second part of a wearable article on each other with an absorptive body interposed between the first part and the second part, the wearable article being included in a continuous material; a conveying step of winding the continuous material including the first part and the second part superposed on each other around an outer peripheral surface of a rotary drum, putting at least a part of the absorptive body into a space between two support faces that are separated from each other on the outer peripheral surface of the rotary drum in a rotation direction of the rotary drum, and conveying the continuous material with the at least part of the absorptive body put into the space by rotation of the rotary drum such that the first part and the second part are supported by the two support faces and the at least part of the absorptive body is disposed inside relative to the two support faces in a radial direction of the rotary drum; and a joining step of moving a movable part of a sealer unit outward and homeward in a cross direction crossing a conveyance direction in which the continuous material is conveyed, with the sealer unit being rotated at a rate identical to a rate of the rotary drum while the continuous material with the at least part of the absorptive body put into the space is conveyed by the rotation of the rotary drum, to form liner sealing areas on an outward path and a homeward path, respectively, and thereby joining the first part and the second part to each other through the formed liner sealing areas.

In the manufacturing method, in the superposing step, the first part corresponding to the front body and the second part corresponding to the back body of the wearable article included in the continuous material are superposed on each other with the absorptive body interposed between the first part and the second part, and in the conveying step, with the at least part of the absorptive body being put into the space formed between the two support faces on the outer peripheral surface of the rotary drum, the continuous material including the first part and the second part superposed on each other is wound around the outer peripheral surface of the rotary drum and is conveyed by rotation of the rotary drum. Then, in the joining step, the first part and the second part are joined together with the at least part of the absorptive body being put into the space such that the at least part of the absorptive body is disposed inside relative to the two support faces in the radial direction of the rotary drum. Thus, in the joining step, the at least part of the absorptive body is put into the space. This reduces the amount of protrusion (raised amount) of either the first part or the second part from the outer peripheral surface of the rotary drum caused by the thickness of the absorptive body. As a result, a peripheral velocity difference between the sealer unit and either the first part or the second part can be reduced. This makes it possible to reduce a discrepancy between the sealing areas on the outward and homeward paths through which the superposed parts of the continuous material are joined to each other.

Preferably, in the method for manufacturing the wearable article, in the conveying step, a whole of the absorptive body is put into the space formed in the outer peripheral surface of the rotary drum, and in the joining step, the first part and the second part are joined together with the whole absorptive body being put in the space.

In the manufacturing method, in the joining step, the whole absorptive body is put into the space in the outer peripheral surface of the rotary drum. This offsets the amount of protrusion of either the first part or the second part from the outer peripheral surface of the rotary drum caused by the thickness of the absorptive body. As a result, a peripheral velocity difference between the sealer unit and either the first part or the second part due to the thickness of the absorptive body can be offset. This makes it possible to reliably reduce a discrepancy between the sealing areas on the outward and homeward paths through which the superposed parts of the continuous material are joined to each other.

Preferably, in the method for manufacturing the wearable article, the space is constituted of a recess formed in the outer peripheral surface of the rotary drum, and a depth of the recess is greater than or equal to a thickness of the absorptive body.

In the manufacturing method, the depth of the recess in the outer peripheral surface of the rotary drum is greater than or equal to the thickness of the absorptive body. Thus, in the joining step, with the whole absorptive body being reliably put into the recess in the outer peripheral surface of the rotary drum and the peripheral velocity difference between the sealer unit and either the first part or the second part being reliably offset, the superposed parts can be joined together. This makes it possible to reduce a discrepancy between the sealing areas with increased reliability.

Preferably, in the method for manufacturing the wearable article, the rotary drum is in a shape of a cylindrical hollow body, and the space is constituted of a through-hole penetrating through a peripheral wall that forms the outer peripheral surface of the rotary drum.

In the manufacturing method, the space is constituted of the through-hole penetrating through the peripheral wall that forms the outer peripheral surface of the rotary drum. Thus, in the joining step, irrespective of the thickness of the absorptive body, with the whole absorptive body being reliably put into the through-hole formed in the outer peripheral surface of the rotary drum and the peripheral velocity difference between the sealer unit and either the first part or the second part being reliably offset, the superposed parts can be joined together. This makes it possible to reliably reduce a discrepancy between the sealing areas irrespective of the thickness of the absorptive body. As a result, the scope of application of this manufacturing method can be widened.

Preferably, in the method for manufacturing the wearable article, the space has a pair of side walls that are separated from each other in a rotation direction of the rotary drum, and the absorptive body put into the space is held by the pair of the side walls in the conveying step and the joining step.

In the manufacturing method, the absorptive body put into the space is held by the pair of the side walls of the space in the conveying step and the joining step. Thus, the peripheral velocity difference between the sealer unit and either the first part or the second part can be reduced to an increased extent. This makes it possible to further reduce a discrepancy between the sealing areas on the outward and homeward paths through which the superposed parts of the continuous material are joined to each other.

The method for manufacturing the wearable article described in the above embodiments has a capability to reduce a discrepancy between sealing areas on outward and homeward paths through which superposed parts of a continuous material are joined to each other when a wearable article that includes an absorptive body is manufactured.

**Claims**

1. A method for manufacturing a wearable article, the method comprising:

   a superposing step of superposing a first part and a second part of a wearable article on each other with an absorptive body interposed between the first part and the second part, the wearable article being included in a continuous material;
   a conveying step of winding the continuous material including the first part and the second part superposed on each other around an outer peripheral surface of a rotary drum, putting at least a part of the absorptive body into a space between two support faces that are separated from each other on the outer peripheral surface of the rotary drum in a rotation direction of the rotary drum, and conveying the continuous material with the at least part of the absorptive body put into the space by rotation of the rotary drum such that the first part and the second part are supported by the two support faces and the at least part of the absorptive body is disposed inside relative to the two support faces in a radial direction of the rotary drum; and
   a joining step of moving a movable part of a sealer unit outward and homeward in a cross direction crossing a conveyance direction in which the continuous material is conveyed, with the sealer unit being rotated at a rate identical to a rate of the rotary drum while the continuous material with the at least part of the absorptive body put into the space is conveyed by the rotation of the rotary drum, to form liner sealing areas on an outward path and a homeward path, respectively, and thereby joining the first part and the second part to each other through the formed liner sealing areas.

2. The method for manufacturing the wearable article according to claim 1, wherein

   in the conveying step, a whole of the absorptive body is put into the space formed in the outer peripheral surface of the rotary drum, and
   in the joining step, the first part and the second part are joined together with the whole absorptive body being put in the space.

3. The method for manufacturing the wearable article according to claim 1 or 2, wherein

   the space is constituted of a recess formed in the outer peripheral surface of the rotary drum, and
   a depth of the recess is greater than or equal to a thickness of the absorptive body.

4.  The method for manufacturing the wearable article according to claim 1 or 2, wherein

    the rotary drum is in a shape of a cylindrical hollow body, and
    the space is constituted of a through-hole penetrating through a peripheral wall that forms the outer peripheral surface of the rotary drum.

5.  The method for manufacturing the wearable article according to any one of claims 1 to 4, wherein

    the space has a pair of side walls that are separated from each other in a rotation direction of the rotary drum, and
    the absorptive body put into the space is held by the pair of the side walls in the conveying step and the joining step.

FIG.1

## FIG.2

FIG.3

EP 4 137 107 A1

# FIG.4

# FIG.5

# FIG.6A

# FIG.6B

FIG.7

# FIG.8A

# FIG.8B

# FIG.9

EP 4 137 107 A1

FIG.10

21

## INTERNATIONAL SEARCH REPORT

| International application No. |
| --- |
| PCT/JP2021/017572 |

A.  CLASSIFICATION OF SUBJECT MATTER
A61F 13/15(2006.01)i
FI: A61F13/15 355B; A61F13/15 357; A61F13/15 370

According to International Patent Classification (IPC) or to both national classification and IPC

B.  FIELDS SEARCHED

Minimum documentation searched (classification system followed by classification symbols)
A61F13/15

Documentation searched other than minimum documentation to the extent that such documents are included in the fields searched
Published examined utility model applications of Japan       1922–1996
Published unexamined utility model applications of Japan     1971–2021
Registered utility model specifications of Japan             1996–2021
Published registered utility model applications of Japan     1994–2021

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X | WO 2015/098535 A1 (ZUIKO CORP.) 02 July 2015 (2015-07-02) paragraphs [0019], [0026]–[0032], [0035]–[0036], [0043], [0046], [0054]–[0057], [0066], [0088]–[0093], fig. 1, 5 | 1 |
| Y | | 2–5 |
| Y | JP 2015-130938 A (UNI-CHARM CORP.) 23 July 2015 (2015-07-23) paragraphs [0035]–[0039], [0043], [0046], [0055], [0068], fig. 2–6 | 1–5 |
| Y | JP 6002867 B1 (UNI-CHARM CORP.) 05 October 2016 (2016-10-05) paragraphs [0057], [0060], fig. 4, 6 | 1–5 |
| Y | JP 2015-85654 A (KAO CORP.) 07 May 2015 (2015-05-07) paragraphs [0015], [0022]–[0023], fig. 5–6 | 1–5 |

☐  Further documents are listed in the continuation of Box C.        ☒  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 09 July 2021 (09.07.2021) | 20 July 2021 (20.07.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2021/017572

| Patent Documents referred in the Report | Publication Date | Patent Family | Publication Date |
|---|---|---|---|
| WO 2015/098535 A1 | 02 Jul. 2015 | CN 105934330 A | |
| JP 2015-130938 A | 23 Jul. 2015 | WO 2015/104880 A1 | |
| | | EP 3092995 A1 | |
| | | paragraphs [0045]-[0050], [0054], [0057], [0069], [0088], fig. 2A-6 | |
| | | CN 105916474 A | |
| JP 6002867 B1 | 05 Oct. 2016 | WO 2017/051476 A1 | |
| JP 2015-85654 A | 07 May 2015 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015098535 A